# EUROPÄISCHE PATENTANMELDUNG

(11) **EP 4 299 281 A1**
(43) Veröffentlichungstag der Anmeldung: **03.01.2024**
(21) Anmeldenummer: 23191993.7
(22) Anmeldetag: 17.08.2023
(51) Int. Cl.: B29C 49/46, B29C 49/12, B29C 49/36, B29C 49/42

(54) **VORRICHTUNG UND VERFAHREN ZUM UMFORMEN VON KUNSTSTOFFVORFORMLINGEN ZU KUNSTSTOFFBEHÄLTNISSEN MIT REINRAUM MIT ABNEHMBARER DECKELEINRICHTUNG**

(30) Priorität: 31.08.2022 DE 102022122085
(71) Anmelder: Krones AG, 93073 Neutraubling (DE)
(72) Erfinder: Roth, Andreas, 93073 Neutraubling (DE); Geltinger, Florian, 93073 Neutraubling (DE); Wittmann, Christian, 93073 Neutraubling (DE); Burgmeier, Mathias, 93073 Neutraubling (DE); Huettner, Christian, 93073 Neutraubling (DE)
(74) Vertreter: Bittner, Bernhard

(57) **Zusammenfassung**

Vorrichtung (1) zum Umformen von Kunststoffvorformlingen (10) zu Kunststoffbehältnissen (15) mit einer ersten Transporteinrichtung (2), welche die umzuformenden Kunststoffvorformlinge entlang eines vorgegebenen Transportpfads transportiert, wobei die Transporteinrichtung (2) einen drehbaren Transportträger (22) aufweist, an dem eine Vielzahl von Umformungsstationen (4) angeordnet ist, wobei diese Umformungsstationen (4) jeweils Blasformeinrichtungen (82) aufweisen, innerhalb derer die Kunststoffvorformlinge (10) durch Beaufschlagung mit einem fließfähigen Medium zu den Kunststoffbehältnissen (15) umformbar sind und die Umformungsstationen (4) jeweils Beaufschlagungseinrichtungen (84) aufweisen, um die Kunststoffvorformlinge (10) mit dem fließfähigen Medium zu beaufschlagen, wobei die Umformungsstationen (4) jeweils Reckeinrichtungen (30) zum Dehnen der Kunststoffvorformlinge in deren Längsrichtung (L) aufweisen und diese Reckeinrichtungen (30) jeweils wenigstens eine in der Längsrichtung (L) der Kunststoffvorformlinge (10) bewegliche Reckstange aufweisen, welche in die Kunststoffvorformlinge einführbar ist und wobei die Vorrichtung einen Reinraum (8) aufweist, innerhalb dessen die Kunststoffvorformlinge (10) zu den Kunststoffbehältnissen expandiert werden sowie eine Dichtungsvorrichtung (50), um den Reinraum (8) gegenüber einer unsterilen Umgebung abzudichten. Erfindungsgemäß ist der Reinraum (8) mit einer Deckeleinrichtung (25) abgeschlossen.

## Beschreibung

Die vorliegende Erfindung bezieht sich auf eine Vorrichtung und ein Verfahren zum Umformen von Kunststoffvorformlingen zu Kunststoffbehältnissen. Derartige Vorrichtungen und Verfahren sind aus dem Stand der Technik seit langem bekannt. Üblicherweise werden dabei erwärmte Kunststoffvorformlinge durch Beaufschlagung mit einem fließfähigen Medium, insbesondere Druckluft, zu Kunststoffbehältnissen umgeformt.

In jüngerer Zeit sind teilweise auch sterile Vorrichtungen dieser Art bekannt geworden. Diese weisen den Vorteil auf, dass Kunststoffvorformlinge entweder bereits unmittelbar aus einem Ofen kommen können und daher steril sind oder aber durch eine Sterilisationseinrichtung sterilisiert wurden. Im Anschluss daran können diese ebenso steril zu den Kunststoffbehältnissen umgeformt werden. Dabei weisen derartige Vorrichtungen üblicherweise Reinräume auf, innerhalb derer der Expansionsvorgang erfolgt.

Im Stand der Technik hat sich gezeigt, dass derartige Vorrichtungen und Verfahren mit diversen technischen Schwierigkeiten verbunden sind, insbesondere was die Größe des jeweiligen Reinraums angeht sowie die Sterilhaltung des Reinraums bzw. dessen Sterilisierung. Hierbei hat sich insbesondere aufgrund der begrenzten Größe des jeweiligen Reinraums ein Einbau, Zusammenbau, Auswechseln oder dgl. von einzelnen Baugruppen der Vorrichtung als relativ umständlich und aufwendig gezeigt.

Ein Paradebeispiel ist hier die Montage von Transporteinrichtungen, die die Kunststoffvorformlinge der Vorrichtung zuführen oder über die die Kunststoffbehältnisse der Vorrichtung abgeführt werden, die zumindest teilweise im Reinraum angeordnet sind. Diese Transporteinrichtungen können aufgrund ihrer eigenen Größe in Verbindung mit der begrenzten Größe des Reinraums nur im Reinraum zusammengebaut werden, sprich eine Vormontage der Transporteinrichtungen ist nicht möglich, was einen außergewöhnlich hohen Zeitaufwand für einen Monteur bzw. Bediener der Vorrichtung bedeutet und darüber hinaus zu erhöhten Taktzeiten am Taktband führt.

Der vorliegenden Erfindung liegt daher die Aufgabe zugrunde, eine montage- und betriebsfreundliche Vorrichtung und Verfahren zum Umformen von Kunststoffvorformlingen zu Kunststoffbehältnissen mit einem Reinraum zur Verfügung zu stellen, bei denen durch den Ein- oder Ausbau bzw. einem Auswechseln von vormontierten Baugruppen an Zeitaufwand gespart werden kann. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Umformen von Kunststoffvorformlingen zu Kunststoffbehältnissen weist eine Transporteinrichtung auf, welche die umzuformenden Kunststoffvorformlinge entlang eines vorgegebenen Transportpfads transportiert. Dabei weist die Transporteinrichtung einen drehbaren Transportträger auf, an dem eine Vielzahl von Umformungsstationen angeordnet ist, wobei diese Umformungsstationen jeweils Blasformeinrichtungen aufweisen, innerhalb derer die Kunststoffvorformlinge durch Beaufschlagung mit einem fließfähigen und insbesondere gasförmigen Medium zu den Kunststoffbehältnissen umformbar und insbesondere expandierbar sind. Bevorzugt verläuft der Transportpfad kreisförmig.

Die Umformungsstationen weisen weiterhin jeweils Beaufschlagungseinrichtungen auf, um die Kunststoffvorformlinge mit dem fließfähigen Medium zu beaufschlagen. Ferner weisen die Umformungsstationen jeweils Reckeinrichtungen zum Dehnen der Kunststoffvorformlinge in deren Längsrichtung auf. Diese Reckeinrichtungen weisen jeweils wenigstens eine in der Längsrichtung der Kunststoffvorformlinge bewegliche Reckstange auf, welche in die Kunststoffvorformlinge einführbar ist.

Weiterhin weist die Vorrichtung einen Reinraum aufweist, innerhalb dessen die Kunststoffvorformlinge zu den Kunststoffbehältnissen expandiert werden oder expandierbar sind sowie eine Dichtungsvorrichtung, um den Reinraum gegenüber einer unsterilen Umgebung abzudichten. Weiterhin weist diese Dichtungseinrichtung wenigstens einen und bevorzugt wenigstens zwei mit einer Flüssigkeit befüllbare oder befüllte umlaufende Kanäle auf.

Erfindungsgemäß ist der Reinraum mit einer Deckeleinrichtung abgeschlossen. Dabei ist diese Deckeleinrichtung derart ausgebildet, dass sie zu Montagezwecken abnehmbar ist. Ein Abnehmen der Deckeleinrichtung im Sinne der vorliegenden Erfindung kann ein teilweises oder vollständigen Entfernern der Deckeleinrichtung von dem Reinraum sein. Unter einem vollständigen Entfernen der Deckeleinrichtung von dem Reinraum ist zu verstehen, dass die Deckeleinrichtung - komplett im Ganzen - abnehmbar ist. Unter einem teilweisen Entfernen der Deckeleinrichtung von dem Reinraum ist zu verstehen, dass die Deckeleinrichtung beispielsweise über entsprechende Scharniere oder dergleichen von dem Reinraum wegschenkbar oder wegklappbar ist.

Bei einer ersten bevorzugten Ausführungsform ist die Deckeleinrichtung lösbar mit dem Reinraum verbunden bzw. die Deckeleinrichtung von dem Reinraum lösbar. Auf diese Weise lässt sich insbesondere der Reinraum öffnen.

Bei einer weiteren bevorzugten Ausführungsform begrenzt die Deckeleinrichtung den Reinraum nach oben hin. Besonders bevorzugt ist die Deckeleinrichtung in einem Dach des Reinraums ausgeführt.

Bei einer weiteren bevorzugten Ausführungsform ist die Deckeleinrichtung als nicht tragende Einrichtung ausgeführt ist.

Unter einer Ausführung als nicht tragende Einrichtung wird insbesondere verstanden, dass die Deckeleinrichtung des Reinraums weder dazu geeignet noch dazu bestimmt ist, Bestandteile der Vorrichtung, wie etwa Antriebe, Umformungsstationen und dergleichen zu tragen.

So können die Deckeleinrichtung beispielsweise in einer Materialstärke und/oder einem Material gefertigt sein, welches keine tragende und/oder stützende Funktion ermöglicht.

Bei einer weiteren bevorzugten Ausführungsform ist die Deckeleinrichtung schwimmend gelagert. Insbesondere kann sie sich daher in vorgegebenen Grenzen in einer horizontalen Richtung bewegen. Dies ist unkritisch, da, wie oben erwähnt, die Deckeleinrichtung bevorzugt nicht als tragende Einrichtung ausgeführt ist.

Bei einer weiteren bevorzugten Ausführungsform weist die die Deckeleinrichtung eine im Wesentlichen kreisförmige oder kreisringförmige Gestalt auf. Desgleichen weist bei einer weiteren bevorzugten Ausführungsform der Reinraum eine im Wesentlichen kreisförmige oder kreisringförmige Ausnehmung auf. Eine bevorzugte Ausführungsform sieht dabei vor, dass die im Wesentlichen kreisförmige oder kreisringförmige Gestalt der Deckeleinrichtung größer ist als die im Wesentlichen kreisförmige oder kreisringförmige Ausnehmung des Reinraums.

Der wichtigste Grund für eine kreisförmige oder kreisringförmige Gestalt der Deckeleinrichtung und des Reinraums hierfür ist, dass die Deckeleinrichtung bei Montagearbeiten nicht in den darunter liegenden Reinraum fallen kann, wenn man die Deckeleinrichtung schräg stellt. Dies liegt an der Kreisform des Deckels. Vom Mittelpunkt aus gemessen sind alle Seiten gleich lang. Stellt man dagegen eine eckige Deckeleinrichtung diagonal auf den Reinraum, fällt diese leicht hinein.

Bei einer weiteren bevorzugten Ausführungsform ist im Bereich der Ausnehmung des Reinraums ein Vorsprung ausgebildet, der dazu geeignet und bestimmt ist, dass die Deckeleinrichtung auf diesem aufliegt. Durch die kreisförmige oder kreisringförmige Gestalt der Deckeleinrichtung liegt diese wesentlich besser in dem Vorsprung des Reinraums als bei einer eckigen Ausführungsform der Deckeleinrichtung, da diese auch nicht klappern.

Besonders bevorzugt ist im Bereich der Ausnehmung des Reinraums ein umlaufender Vorsprung ausgebildet, ähnlich einem Rahmen. Alternativ kann der Vorsprung auch stiftartig, kegelartig oder dergleichen im Bereich der Ausnehmung des Reinraums ausgebildet sein. Daneben können jedoch auch jegliche andere Aufnahme- bzw. Auflageelemente im Bereich der Ausnehmung des Reinraums ausgebildet sind, die dazu geeignet und bestimmt sind, dass die Deckeleinrichtung auf diesen aufliegt.

Bei einer weiteren bevorzugten Ausführungsform sind die Kunststoffvorformlinge über eine zweite Transporteinrichtung der Vorrichtung zuführbar und über eine dritte Transporteinrichtung der Vorrichtung abtransportierbar. Vorteilhaft handelt es sich somit bei der zweiten Transporteinrichtung um eine Zuführeinrichtung und bei der dritten Transporteinrichtung um eine Abführeinrichtung.

Bevorzugt ist die zweite Transporteinrichtung wenigstens abschnittsweise innerhalb des Reinraums angeordnet. Ebenso ist bevorzugt die dritte Transporteinrichtung wenigstens abschnittsweise innerhalb des Reinraums angeordnet. Vorteilhaft ist die zweite Transporteinrichtung als tragende Einrichtung ausgeführt. Ebenso ist die dritte Transporteinrichtung vorteilhaft als tragende Einrichtung ausgeführt. Besonders bevorzugt sind sowohl die zweite als auch die dritte Transporteinrichtung, insbesondere sowohl die Zuführ- als auch die Abführeinrichtung, als tragende Einrichtung ausgeführt.

Bei einer weiteren bevorzugten Ausführungsform sind die erste und/oder zweite Transporteinrichtung über die Ausnehmung des Reinraums einsetz- bzw. rausnehmbar.

Eine besonders bevorzugte Ausführungsform sieht dabei vor, dass die erste und/oder zweite Transporteinrichtung als Transportsterne ausgebildet sind.

Bevorzugt ist der Transportstern der zweiten Transporteinrichtung ein Teilungsverzugsstern handeln.

Eine weitere besonders bevorzugte Ausführungsform sieht dabei vor, dass die Transportsterne als vormontierte Transportsterne über die Ausnehmung des Reinraums einsetz- bzw. rausnehmbar sind.

Durch die zuvor beschriebene Vorrichtung können jegliche vormontierte Baugruppen, vorzugsweise Transportsterne, ganz einfach und unkompliziert über die Ausnehmung des Reinraums eingesetzt bzw. rausgenommen werden. Auch kann durch diesen Ein- oder Ausbau bzw. durch das Auswechseln von vormontierten Baugruppen, besonders bevorzugt vormontierten Transportsternen, an Zeitaufwand gespart werden, da die Transportsterne ganz einfach im Ganzen auf dem Taktband montiert werden können, anstelle des vormals aus dem Stand der Technik bekannten und zugleich aufwendigen und komplizierten Zusammenbaus der Transportsterne im Reinraum.

Die vorliegende Erfindung ist weiterhin auf ein Verfahren zum Umformen von Kunststoffvorformlingen zu Kunststoffbehältnissen gerichtet, wobei eine Transporteinrichtung die umzuformenden Kunststoffvorformlinge entlang eines vorgegebenen Transportpfads transportiert und wobei die Transporteinrichtung einen drehbaren Transportträger aufweist, an dem eine Vielzahl von Umformungsstationen angeordnet wird oder angeordnet ist, wobei diese Umformungsstationen jeweils Blasformeinrichtungen aufweisen, innerhalb derer die Kunststoffvorformlinge durch Beaufschlagung mit einem fließfähigen Medium zu den Kunststoffbehältnissen umgeformt werden und die Umformungsstationen jeweils Beaufschlagungseinrichtungen aufweisen, welche die Kunststoffvorformlinge mit dem fließfähigen Medium und insbesondere gasförmigen Medium (und insbesondere Sterilluft) beaufschlagen, wobei die Umformungsstationen jeweils Reckeinrichtungen zum Dehnen der Kunststoffvorformlinge in deren Längsrichtung aufweisen bzw. Reckeinrichtungen, welche die Kunststoffvorformlinge in deren Längsrichtung dehnen und diese Reckeinrichtungen jeweils wenigstens eine in der Längsrichtung der Kunststoffvorformlinge bewegliche und/oder sich bewegende Reckstange aufweisen, welche in die Kunststoffvorformlinge eingeführt wird.

Weiterhin weist die Vorrichtung einen Reinraum auf, innerhalb dessen die Kunststoffvorformlinge zu den Kunststoffbehältnissen expandiert werden sowie eine Dichtungsvorrichtung, welche den Reinraum gegenüber einer unsterilen Umgebung abdichtet. Dabei weist die Dichtungseinrichtung bevorzugt wenigstens einen mit einer Flüssigkeit befüllten umlaufenden Kanal auf.

Erfindungsgemäß wird der Reinraum mit einer Deckeleinrichtung abgeschlossen. Diese Deckeleinrichtung ist dabei derart ausgebildet, dass sie zu Montagezwecken abnehmbar ist.

Dabei ist die oben beschriebene Vorrichtung insbesondere dazu eingerichtet und dafür vorgesehen, dieses beschriebene Verfahren durchzuführen, d. h., dass alle für die obig beschriebene Vorrichtung ausgeführten Merkmale ebenso für das hier beschriebene Verfahren offenbart sind und umgekehrt.

Weitere Vorteile und Ausführungsformen ergeben sich aus der beigefügten Zeichnung.

Darin zeigen:
- Fig. 1: eine schematische Darstellung einer erfindungsgemäßen Vorrichtung; und
- Fig. 2: zeigt eine teilweise Ansicht des Reinraums 8 der erfindungsgemäßen Vorrichtung.

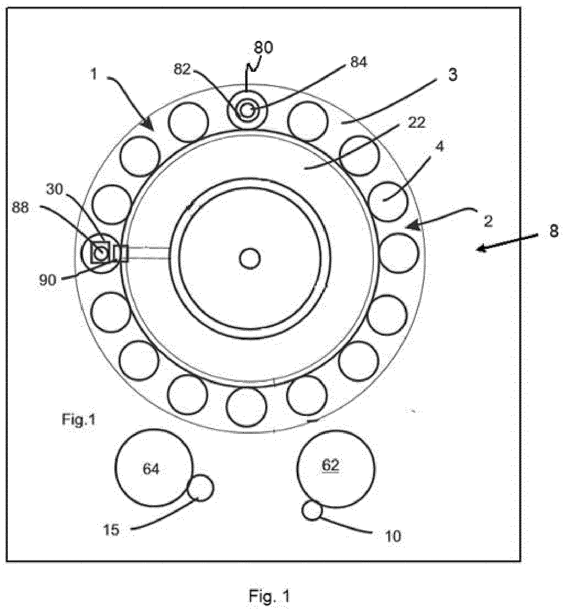

Figur 1 zeigt eine Vorrichtung 1 zum Umformen von Kunststoffvorformlingen 10 zu Kunststoffbehältnissen 15. Diese Vorrichtung weist eine Umformungseinrichtung 3 mit einer ersten Transporteinrichtung 2 mit einem drehbaren Träger 22 auf, an dem eine Vielzahl von Umformungsstationen 4 angeordnet ist. Diese einzelnen Umformungsstationen 4 weisen jeweils Blasformeinrichtungen 82 auf, die in ihrem Inneren einen Hohlraum zum Expandieren der Kunststoffvorformlinge 10 ausbilden.

Das Bezugszeichen 84 kennzeichnet eine Beaufschlagungseinrichtung, welche zum Expandieren der Kunststoffvorformlinge 10 dient. Hierbei kann es sich beispielsweise um eine Blasdüse handeln, welche an eine Mündung der Kunststoffvorformlinge 10 anlegbar ist, um diese so zu expandieren. Daneben wäre es auch denkbar, dass die Blasdüse an der Blasform und/oder einem Tragring des Kunststoffvorformlings 10 und/oder im Inneren der Mündung des Kunststoffvorformlings 10 und/oder am Mündungsrand des Kunststoffvorformlings 10 abdichtet.

Das Bezugszeichen 90 kennzeichnet eine Ventilanordnung wie einen Ventilblock, der bevorzugt eine Vielzahl von Ventilen aufweist, welche die Beaufschlagung der Kunststoffvorformlinge 10 mit unterschiedlichen Druckniveaus steuern.

Das Bezugszeichen 88 kennzeichnet eine Reckstange, die dazu dient, die Kunststoffvorformlinge in ihrer Längsrichtung zu dehnen. Bevorzugt weisen sämtliche Umformungsstationen 4 derartige Blasformen 82, Beaufschlagungseinrichtungen 84 sowie Reckstangen 88 auf. Diese Reckstange ist bevorzugt Bestandteil einer mit 30 bezeichneten Reckeinrichtung.

Bevorzugt liegt die Anzahl dieser Umformungsstationen 4 zwischen 2 und 100, bevorzugt zwischen 4 und 60, bevorzugt zwischen 6 und 40.

Die Kunststoffvorformlinge 10 werden über eine zweite Transporteinrichtung 62 der Vorrichtung 1 zugeführt. Über eine dritte Transporteinrichtung 64 werden die Kunststoffbehältnisse 15 abtransportiert. So handelt es sich vorzugsweise bei der zweiten Transporteinrichtung 62 um eine Zuführeinrichtung und bei der dritten Transporteinrichtung 64 um eine Abführeinrichtung. Dabei sind die erste und/oder zweite Transporteinrichtung 62, 64 bevorzugt als Transportsterne ausgebildet.

Das Bezugszeichen 8 kennzeichnet einen Reinraum, der bevorzugt so ausgebildet ist, dass er zumindest den Transportpfad der Kunststoffvorformlinge 10 umgibt. In dieser schematischen Darstellung ist der Reinraum 8 lediglich schematisch als Rechteck dargestellt. Bevorzugt ist der Reinraum 8 mit einer Dichtungseinrichtung, welche bevorzugt wenigstens ein Wasserschloss aufweist, gegenüber der unsterilen Umgebung abgedichtet.

Bevorzugt sind auch Elemente der zweiten Transporteinrichtung 62 und der dritten Transporteinrichtung 64 innerhalb des Reinraums 8 angeordnet. Insbesondere umgibt der Reinraum 8 bevorzugt auch im Bereich der zweiten und dritten Transporteinrichtung 62, 64 den Transportpfad der Kunststoffvorformlinge 10 bzw. Kunststoffbehältnisse 15.

Weiterhin weist die Vorrichtung 1 eine (in Fig. 1 nicht gezeigte) Deckeleinrichtung 25 auf, welche den Reinraum 8 nach oben hin begrenzt.

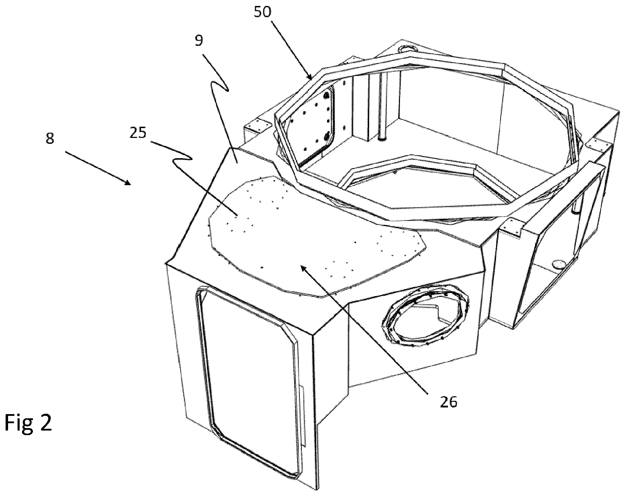

Figur 2 zeigt eine Ansicht des Reinraums 8 der erfindungsgemäßen Vorrichtung 1 (s. hierzu Fig. 1), der mit einer entsprechend der Ausnehmung 26 ausgebildeten Deckleinrichtung 25 abschließbar ist.

Wie hier dargestellt ist, ist die Deckeleinrichtung 25 vorzugsweise in einem Dach 9 des Reinraums 8 ausgeführt und begrenzt den Reinraum 8 nach oben hin. Der Reinraum 8 und die Deckeleinrichtung 25 weisen eine im Wesentlichen kreisförmige oder kreisringförmige Gestalt bzw. Ausnehmung 26 auf. Dabei ist die im Wesentlichen kreisförmige oder kreisringförmige Gestalt der Deckeleinrichtung 25 größer als die im Wesentlichen kreisförmige oder kreisringförmige Ausnehmung 26 des Reinraums 8.

Dabei kann die Deckeleinrichtung 25, wie in Fig. 2 nicht dargestellt ist, vollständig entfernt sein, d. h. die Deckeleinrichtung 25 ist von dem Reinraum 8 abgenommen. Erfindungsgemäß ist die Deckeleinrichtung 25 derart ausgebildet, dass sie zu Montagezwecken abnehmbar ist, sprich die Deckeleinrichtung 25 ist bevorzugt lösbar mit dem Reinraum 8 verbunden bzw. die Deckeleinrichtung 25 ist von dem Reinraum 8 lösbar. Auf diese Weise lässt sich der Reinraum 8 öffnen.

Wie hier dargestellt, ist im Bereich der Ausnehmung 26 des Reinraums 8 ein Vorsprung ausgebildet, der dazu geeignet und bestimmt ist, dass die Deckeleinrichtung 25 auf diesem aufliegt. Der Vorsprung ist hier im Bereich der Ausnehmung 26 des Reinraums 8 als ein umlaufender Vorsprung ausgebildet. Andere Ausführungsformen können auch anderweitige Vorsprünge oder Aufnahme- bzw. Auflageelemente im Bereich der Ausnehmung 26 des Reinraums 8 aufweisen, solange sie ermöglichen, dass die Deckeleinrichtung 25 auf ihnen aufliegen kann.

So sind die in Fig. 1 dargestellten vorzugsweise als vormontierte Transportsterne ausgebildete erste und/oder zweite Transporteinrichtung 62, 64 ganz einfach und unkompliziert über die Ausnehmung 26 des Reinraums 8 einsetz- bzw. rausnehmbar. Im Allgemeinen können jegliche vormonierten Baugruppen über diese Ausnehmung 26 des Reinraums 8 in den Reinraum 8 eingesetzt bzw. rausgenommen werden. Damit ist eine enorme Zeitersparnis gegenüber dem vormaligen Zusammenbau der Baugruppen im Reinraum 8 erzielt.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Vorrichtung
- 2: erste Transporteinrichtung
- 3: Umformungseinrichtung
- 4: Umformungsstation
- 8: Reinraum
- 9: Dach
- 10: Kunststoffvorformling
- 15: Kunststoffbehältnis
- 22: Transportträger
- 25: Deckeleinrichtung
- 26: Ausnehmung
- 30: Reckeinrichtung
- 50: Dichtungsvorrichtung
- 62: zweite Transporteinrichtung, Transportstern
- 64: dritte Transporteinrichtung, Transportstern
- 80: Blasform
- 82: Blasformeinrichtung
- 84: Beaufschlagungseinrichtung
- 88: Reckstange
- 90: Ventilanordnung

- L: Längsrichtung

## Patentansprüche

1. Vorrichtung (1) zum Umformen von Kunststoffvorformlingen (10) zu Kunststoffbehältnissen (15) mit einer ersten Transporteinrichtung (2), welche die umzuformenden Kunststoffvorformlinge entlang eines vorgegebenen Transportpfads transportiert, wobei die Transporteinrichtung (2) einen drehbaren Transportträger (22) aufweist, an dem eine Vielzahl von Umformungsstationen (4) angeordnet ist, wobei diese Umformungsstationen (4) jeweils Blasformeinrichtungen (82) aufweisen, innerhalb derer die Kunststoffvorformlinge (10) durch Beaufschlagung mit einem fließfähigen Medium zu den Kunststoffbehältnissen (15) umformbar sind und die Umformungsstationen (4) jeweils Beaufschlagungseinrichtungen (84) aufweisen, um die Kunststoffvorformlinge (10) mit dem fließfähigen Medium zu beaufschlagen, wobei die Umformungsstationen (4) jeweils Reckeinrichtungen (30) zum Dehnen der Kunststoffvorformlinge in deren Längsrichtung (L) aufweisen und diese Reckeinrichtungen (30) jeweils wenigstens eine in der Längsrichtung (L) der Kunststoffvorformlinge (10) bewegliche Reckstange (88) aufweisen, welche in die Kunststoffvorformlinge einführbar ist und wobei die Vorrichtung einen Reinraum (8) aufweist, innerhalb dessen die Kunststoffvorformlinge (10) zu den Kunststoffbehältnissen expandiert werden sowie eine Dichtungsvorrichtung (50), um den Reinraum (8) gegenüber einer unsterilen Umgebung abzudichten, **dadurch gekennzeichnet, dass**
der Reinraum (8) mit einer Deckeleinrichtung (25) abgeschlossen ist und diese Deckeleinrichtung (25) derart ausgebildet ist, dass sie zu Montagezwecken abnehmbar ist.

2. Vorrichtung (1) nach wenigstens einem der vorangegangenen Ansprüche,
**dadurch gekennzeichnet, dass**
die Deckeleinrichtung (25) lösbar mit dem Reinraum (8) verbunden ist.

3. Vorrichtung (1) nach wenigstens einem der vorangegangenen Ansprüche,
**dadurch gekennzeichnet, dass** die Deckeleinrichtung (25) den Reinraum (8) nach oben hin begrenzt, und vorzugsweise im Dach (9) des Reinraums (8) ausgeführt ist.

4. Vorrichtung nach wenigstens einem der vorangegangenen Ansprüche,
**dadurch gekennzeichnet, dass**
die Deckeleinrichtung (25) als nicht tragende Einrichtung ausgeführt ist.

5. Vorrichtung (1) nach wenigstens einem der vorangegangenen Ansprüche,
**dadurch gekennzeichnet, dass**
die Deckeleinrichtung (25) schwimmend gelagert ist.

6. Vorrichtung (1) nach wenigstens einem der vorangegangenen Ansprüche,
**dadurch gekennzeichnet, dass**
die Deckeleinrichtung (25) eine im Wesentlichen kreisförmige oder kreisringförmige Gestalt aufweist.

7. Vorrichtung (1) nach wenigstens einem der vorangegangenen Ansprüche,
**dadurch gekennzeichnet, dass**
der Reinraum (8) eine im Wesentlichen kreisförmige oder kreisringförmige Ausnehmung (26) aufweist.

8. Vorrichtung (1) nach wenigstens einem der vorangegangenen Ansprüche,
**dadurch gekennzeichnet, dass**
die im Wesentlichen kreisförmige oder kreisringförmige Gestalt der Deckeleinrichtung (25) größer ist als die im Wesentlichen kreisförmige oder kreisringförmige Ausnehmung (26) des Reinraums (8).

9. Vorrichtung (1) nach wenigstens einem der vorangegangenen Ansprüche,
**dadurch gekennzeichnet, dass**
im Bereich der Ausnehmung (26) des Reinraums (8) ein Vorsprung ausgebildet ist, der dazu geeignet und bestimmt ist, dass die Deckeleinrichtung (25) auf diesem aufliegt.

10. Vorrichtung (1) nach wenigstens einem der vorangegangenen Ansprüche,
**dadurch gekennzeichnet, dass**
die Kunststoffvorformlinge (10) über eine zweite Transporteinrichtung (62) der Vorrichtung (1) zuführbar und über eine dritte Transporteinrichtung (64) der Vorrichtung (1) die Kunststoffbehältnisse (15) abtransportierbar sind.

11. Vorrichtung (1) nach wenigstens einem der vorangegangenen Ansprüche,
**dadurch gekennzeichnet, dass**
die zweite und/oder dritte Transporteinrichtung (62, 64) über die Ausnehmung (26) des Reinraums (8) einsetz- bzw. rausnehmbar sind.

12. Vorrichtung (1) nach wenigstens einem der vorangegangenen Ansprüche,
**dadurch gekennzeichnet, dass**
die zweite und/oder dritte Transporteinrichtung (62, 64) als Transportsterne (62, 64) ausgebildet sind.

13. Vorrichtung (1) nach Anspruch 10,
**dadurch gekennzeichnet, dass**
die Transportsterne als vormontierte Transportsterne (62, 64) über die Ausnehmung (26) des Reinraums (8) einsetz- bzw. rausnehmbar sind.

14. Vorrichtung (1) nach wenigstens einem der vorangegangenen Ansprüche,
**dadurch gekennzeichnet, dass**
die Deckeleinrichtung (25) lösbar mit dem Reinraum (8) verbunden ist.

15. Verfahren zum Umformen von Kunststoffvorformlingen (10) zu Kunststoffbehältnissen (15), wobei eine erste Transporteinrichtung (2), die umzuformenden Kunststoffvorformlinge entlang eines vorgegebenen Transportpfads transportiert, wobei die Transporteinrichtung (2) einen drehbaren Transportträger (22) aufweist, an dem eine Vielzahl von Umformungsstationen (4) angeordnet ist, wobei diese Umformungsstationen (4) jeweils Blasformeinrichtungen aufweisen, innerhalb derer die Kunststoffvorformlinge (10) durch Beaufschlagung mit einem fließfähigen Medium zu den Kunststoffbehältnissen (15) umgeformt werden und die Umformungsstationen (4) jeweils Beaufschlagungseinrichtungen (40) aufweisen, welche die Kunststoffvorformlinge (10) mit dem fließfähigen Medium beaufschlagen, wobei die Umformungsstationen (4) jeweils Reckeinrichtungen (30) zum Dehnen der Kunststoffvorformlinge in deren Längsrichtung (L) aufweisen und diese Reckeinrichtungen (30) jeweils wenigstens eine in der Längsrichtung (L) der Kunststoffvorformlinge (10) bewegliche Reckstange aufweisen, welche in die Kunststoffvorformlinge eingeführt wird und wobei die Vorrichtung einen Reinraum (8) aufweist, innerhalb dessen die Kunststoffvorformlinge (10) zu den Kunststoffbehältnissen expandiert werden sowie eine Dichtungsvorrichtung (50), welche den Reinraum (8) gegenüber einer unsterilen Umgebung abdichtet,
**dadurch gekennzeichnet, dass**
der Reinraum (8) mit einer Deckeleinrichtung (25) abgeschlossen wird und diese Deckeleinrichtung (25) derart ausgebildet ist, dass sie zu Montagezwecken abnehmbar ist.
